# EUROPEAN PATENT APPLICATION

(11) **EP 4 660 198 A1**
(43) Date of publication of application: **10.12.2025**
(21) Application number: 23919206.5
(22) Date of filing: 26.04.2023
(51) Int. Cl.: C07H 3/02, C07H 1/06

(54) **METHOD FOR CONTROLLING IMPURITIES IN L-ARABINOSE AND USE THEREOF**

(30) Priority: 03.02.2023 CN 202310055013
(71) Applicant: THOMSON BIOTECH (XIAMEN) PTE. LTD., Xiamen, Fujian 361026 (CN)
(72) Inventor: LIN, Weijun, Xiamen, Fujian 361026 (CN); SHI, Guoliang, Xiamen, Fujian 361026 (CN); CAI, Xubin, Xiamen, Fujian 361026 (CN); XIAO, Yongcheng, Xiamen, Fujian 361026 (CN); ZHANG, Lei, Xiamen, Fujian 361026 (CN); XIAO, Shunzhi, Xiamen, Fujian 361026 (CN); YANG, Yong, Nanjing, Jiangsu 211198 (CN); CAO, Feng, Nanjing, Jiangsu 211100 (CN)
(74) Representative: Gille Hrabal Partnerschaftsgesellschaft mbB Patentanwälte
(86) International application number: PCT/CN2023/090906
(87) International publication number: WO 2024/159638

(57) **Abstract**

The present invention relates to a method for controlling impurities in L-arabinose and the use thereof. The method comprises: removing impurities in a starting raw material of L-arabinose by means of using adsorption resin. According to the method and the use provided in the present invention, the content of impurities such as 5-hydroxymethylfurfural and furfural in the L-arabinose product can be effectively controlled, and particularly, impurities can be removed from the source and upstream process, so that the toxic side effects of impurities are reduced, and the quality and the administration safety of the L-arabinose product are improved.

## Description

The present application claims priority to the prior application with the Patent Application No. 202310055013.8 entitled "METHOD FOR CONTROLLING IMPURITIES IN L-ARABINOSE AND USE THEREOF" and filed with the China National Intellectual Property Administration on Feb. 3, 2023, which is incorporated herein by reference in its entirety.

### TECHNICAL FIELD

The present disclosure relates to the field of saccharide production processes, and in particular to a method for controlling impurities in L-arabinose and use thereof.

### BACKGROUND

L-Arabinose (C₃H₁₀O₅), also known as L(+)-arabinose, is an aldopentose. It is a monosaccharide originally isolated from gum arabic. Research shows that L-arabinose has the effects of assisting in lowering blood sugar and losing weight, and the good functions of moistening the intestine and relieving constipation.

L-Arabinose is mainly derived from lignocellulosic biomass, such as corn, sugar cane, sugar beet, and other plants. Literature data show that L-arabinose undergoes dehydration and degradation under acidic and high-temperature conditions, producing the mutagenic impurity furfural, and the degradation pathway is shown in the following figure:

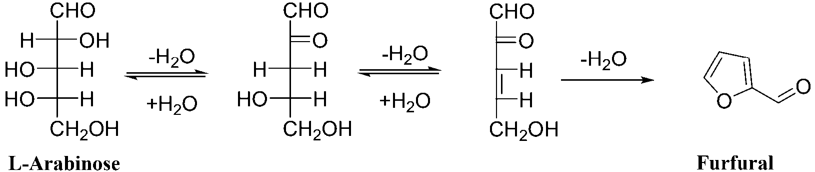

On October 27, 2017, the International Agency for Research on Cancer of the World Health Organization released a preliminary consolidated reference list of carcinogens, and furfural was a mutagenic impurity in the Group 3 carcinogen list.

5-Hydroxymethylfurfural (5-HMF) exhibits nephrotoxicity, mutagenicity, and potential genotoxicity. Pharmacopoeias explicitly define 5-HMF as a restricted component in glucose injectable liquids. However, for L-arabinose formulations, there remains a lack of in-depth research on the related content and control of 5-HMF.

In order to improve the quality and intake safety of various L-arabinose products, it is imperative to effectively control the content of impurities such as 5-hydroxymethylfurfural, furfural, and the like at the source and during upstream production processes to reduce the risk of toxic and side effects of the impurities, thereby facilitating the large-scale industrial production of L-arabinose products and their further application in food and pharmaceutical fields.

### SUMMARY

In order to solve the problems in the prior art, in a first aspect, the present disclosure provides a method for controlling impurities, which comprises:
(I-a) removing impurities in an L-arabinose starting raw material by using an adsorption resin.

According to an embodiment of the present disclosure, the impurities are selected from at least one of furfural and 5-hydroxymethylfurfural.

According to an embodiment of the present disclosure, the adsorption resin is selected from at least one of a macroporous adsorption resin with high specific surface area, a styrenic macroporous resin, and a styrene-divinylbenzene copolymer skeleton macroporous resin.

In some embodiments, the adsorption resin is selected from at least one of a styrenic macroporous strong acid cation exchange resin and a styrenic macroporous weak base anion exchange resin.

In some embodiments, the adsorption resin is selected from a sulfonic acid-based cation exchange resin, such as a cation exchange resin having a sulfonic acid group (-SO₃H) on a styrene-divinylbenzene copolymer.

According to an embodiment of the present disclosure, the styrene-divinylbenzene copolymer skeleton macroporous resin is selected from weak polarity and moderate polarity.

According to an embodiment of the present disclosure, the adsorption resin is selected from at least one of LSA-220, D309, SQD-96, D301-M, D301, D319, LSA-20, LX-10B, LX-100B, LX-20B, LX-360, LX-150, LSA-10, LSA-12, LSA-10B, LSA-100, LSA-210, HPD296, HPD500, HPD300L, D-900, and 001×7. Preferably, the resin is selected from at least one of LSA-220, SQD-96, D319, LSA-20, and LX-10B; more preferably, the resin is selected from at least one of LSA-220 and SQD-96. More preferably, the adsorption resin is selected from a combination of D301 and 001×7.

In some embodiments, the L-arabinose starting raw material is first adsorbed by a D301 resin, then adsorbed by a 001×7 resin, and finally adsorbed by a mixed resin of D301 and 001×7; or the L-arabinose starting raw material is first adsorbed by a 001×7 resin, then adsorbed by a D301 resin, and finally adsorbed by a mixed resin of D301 and 001×7; in the mixed resin, a mixing volume ratio of D301 to 001×7 is 1:1 to 3:1, such as 2:1.

In some embodiments, the L-arabinose starting raw material is first adsorbed by a D301 resin, then adsorbed by a 001×7 resin, then adsorbed by a mixed resin of D301 and 001×7, and finally adsorbed by LSA-220; or the L-arabinose starting raw material is first adsorbed by a 001×7 resin, then adsorbed by a D301 resin, then adsorbed by a mixed resin of D301 and 001×7, and finally adsorbed by LSA-220; in the mixed resin, a mixing volume ratio of D301 to 001×7 is 1:1 to 3:1, such as 2:1; a volume ratio of D301:001×7:the mixed resin = (1-3):1:(1-3), such as 2:1:2.

According to an embodiment of the present disclosure, the adsorption resin is purchased from Sunresin or Suqing.

According to an embodiment of the present disclosure, the resin is selected from at least one of Sunresin LSA-220, Suqing D309, Suqing SQD-96, Suqing D301-M, Suqing D301, Suqing D319, Sunresin LSA-20, Sunresin LX-10B, and Suqing 001×7. Preferably, the resin is selected from at least one of Sunresin LSA-220, Suqing SQD-96, Suqing D319, Sunresin LSA-20, Sunresin Lx-10B, and Suqing 001×7; more preferably, the resin is selected from at least one of Sunresin LSA-220 and Suqing SQD-96.

In some embodiments, the L-arabinose starting raw material is first adsorbed by a Suqing D301 resin, then adsorbed by a Suqing 001×7 resin, and finally adsorbed by a mixed resin of Suqing D301 and Suqing 001×7; or the L-arabinose starting raw material is first adsorbed by a Suqing 001×7 resin, then adsorbed by a Suqing D301 resin, and finally adsorbed by a mixed resin of Suqing D301 and Suqing 001×7; in the mixed resin, a mixing volume ratio of Suqing D301 to Suqing 001×7 is 1:1 to 3:1, such as 2:1. A volume ratio of the Suqing D301 resin:Suqing 001×7:the mixed resin = (1-3): 1:(1-3), such as 2:1:2.

In some embodiments, the L-arabinose starting raw material is first adsorbed by a Suqing D301 resin, then adsorbed by a Suqing 001×7 resin, then adsorbed by a mixed resin of Suqing D301 and Suqing 001×7, and finally adsorbed by LSA-220; or the L-arabinose starting raw material is first adsorbed by a Suqing 001×7 resin, then adsorbed by a Suqing D301 resin, then adsorbed by a mixed resin of Suqing D301 and Suqing 001×7, and finally adsorbed by LSA-220; in the mixed resin, a mixing volume ratio of Suqing D301 to Suqing 001×7 is 1:1 to 3:1, such as 2:1. A volume ratio of the Suqing D301 resin:Suqing 001×7:the mixed resin = (1-3):1:(1-3), such as 2:1:2.

According to an embodiment of the present disclosure, in step (I-a), the L-arabinose starting raw material may be selected from an L-arabinose solid raw material, an L-arabinose-containing syrup, and an L-arabinose-containing formulation.

In some embodiments, the L-arabinose starting raw material is selected from a commercially available or synthesized L-arabinose solid. For example, the purity of the L-arabinose in the solid is ≥90%.

In some embodiments, the L-arabinose starting raw material is selected from an L-arabinose-containing liquid crude product extracted from natural products and a crystallization mother liquor in a purification process of an L-arabinose-containing syrup.

In some embodiments, the L-arabinose starting raw material is selected from an L-arabinose-containing syrup. The L-arabinose-containing syrup may be selected from commercially available products.

In some embodiments, a sugar degree of the L-arabinose-containing syrup is 20-90% Brix, for example, the sugar degree is 20% Brix, 25% Brix, 30% Brix, 35% Brix, 40% Brix, 45% Brix, 50% Brix, 55% Brix, 60% Brix, 65% Brix, 70% Brix, 75% Brix, 80% Brix, 85% Brix, or 90% Brix.

In some embodiments, the L-arabinose starting raw material is selected from an L-arabinose-containing aqueous solution. A sugar degree of the L-arabinose-containing aqueous solution is 20-90% Brix, for example, the sugar degree is 20% Brix, 25% Brix, 30% Brix, 35% Brix, 40% Brix, 45% Brix, 50% Brix, 55% Brix, 60% Brix, 65% Brix, 70% Brix, 75% Brix, 80% Brix, 85% Brix, or 90% Brix; or in the L-arabinose-containing aqueous solution, a mass concentration of L-arabinose is ≥10%, for example, the mass concentration of L-arabinose is ≥10%, ≥15%, ≥20%, ≥25%, ≥30%, ≥35%, ≥40%, ≥45%, ≥50%, ≥55%, ≥60%, ≥65%, ≥70%, ≥75%, ≥80%, ≥85%, or ≥90%.

More specifically, in some embodiments, in the L-arabinose-containing syrup, a mass concentration of L-arabinose is ≥10%, for example, the mass concentration of L-arabinose is ≥10%, ≥15%, ≥20%, ≥25%, ≥30%, ≥35%, ≥40%, ≥45%, ≥50%, ≥55%, ≥60%, ≥65%, ≥70%, ≥75%, ≥80%, ≥85%, or ≥90%. In some embodiments, the L-arabinose starting raw material is selected from any L-arabinose-containing formulation, for example, the formulation may be a liquid formulation, and in the liquid formulation, a mass concentration of L-arabinose is ≥10%, ≥15%, ≥20%, ≥25%, ≥30%, ≥35%, ≥40%, ≥45%, 50%, ≥55%, ≥60%, ≥65%, ≥70%, ≥75%, ≥80%, ≥85%, or ≥90%. The formulation may contain L-arabinose as the sole active ingredient or may contain an additional active ingredient.

According to an embodiment of the present disclosure, in step (I-a), in the L-arabinose starting raw material, a content of furfural is more than 10000 ng/mL, such as more than 10000 ng/mL, 20000 ng/mL, 30000 ng/mL, 40000 ng/mL, 50000 ng/mL, 60000 ng/mL, 70000 ng/mL, 80000 ng/mL, 90000 ng/mL, 100000 ng/mL, 110000 ng/mL, 120000 ng/mL, 130000 ng/mL, 140000 ng/mL, or 150000 ng/mL; a content of 5-hydroxymethylfurfural is more than 500 ng/mL, such as more than 500 ng/mL, 1000 ng/mL, 2000 ng/mL, 3000 ng/mL, 4000 ng/mL, 5000 ng/mL, 6000 ng/mL, 7000 ng/mL, 8000 ng/mL, 9000 ng/mL, 10000 ng/mL, 15000 ng/mL, 20000 ng/mL, 30000 ng/mL, 40000 ng/mL, or 50000 ng/mL. In some embodiments, in the L-arabinose starting raw material, the content of furfural is more than 50000 ng/mL. In some embodiments, in the L-arabinose starting raw material, the content of 5-hydroxymethylfurfural is more than 10000 ng/mL.

According to an embodiment of the present disclosure, in step (I-a), a sugar degree of the L-arabinose starting raw material is ≤60% Brix when passing through a column, such as 30% Brix, 35% Brix, 40% Brix, 45% Brix, 50% Brix, or 55% Brix; preferably, the sugar degree is ≤50% Brix. More preferably, the sugar degree is ≤40% Brix. Optionally, step (I-a) comprises a step of dissolving or diluting the L-arabinose starting raw material with water to a suitable sugar degree for passing through the column.

According to an embodiment of the present disclosure, in step (I-a), a column flow rate for the material is 0.5 BV/h to 4 BV/h, such as 0.5 BV/h, 1.0 BV/h, 1.5 BV/h, 2.0 BV/h, 2.5 BV/h, 3.0 BV/h, or 4.0 BV/h; the column temperature is 10-50 °C, preferably room temperature (25±5 °C); an elution solvent used is water.

In some embodiments, step (I-a) is as follows: passing a raw material syrup at a rate of 1-3 BV/h sequentially through a Suqing D301 resin, a 001×7 resin, a mixed resin (a volume ratio of D301 to 001×7 is 2:1), and then Sunresin LSA-220, wherein a volume ratio of the Suqing D301 resin:Suqing 001×7:the mixed resin = (1-3):1:(1-3), such as 2:1:2.

According to an embodiment of the present disclosure, the method may further comprise step (I-b) of activated carbon treatment. Step (I-b) may be performed before step (I-a) or may be performed after step (I-a).

According to an embodiment of the present disclosure, in step (I-b), the activated carbon is selected from powdered activated carbon and granular activated carbon. An adsorption temperature of the activated carbon is 20-80 °C, such as 30 °C, 35 °C, 40 °C, 45 °C, 50 °C, 55 °C, 60 °C, 65 °C, 70 °C, or 75 °C; an adsorption time of the selected activated carbon is 10-100 min, preferably 20-90 min, such as 30 min, 40 min, 50 min, 60 min, 70 min, 80 min, or 90 min. An amount of the activated carbon used is as follows: 0.0005-0.010 g of the activated carbon is used for adsorbing every 1 mL of the material, for example, 0.0005 g, 0.001 g, 0.002 g, 0.003 g, 0.004 g, 0.005 g, 0.006 g, 0.007 g, 0.008 g, 0.009 g, or 0.010 g of the activated carbon is used.

In some embodiments, step (I-b) is as follows: warming the syrup obtained in step (I-a) to 40-60 °C, adding activated carbon for adsorption, and performing suction filtration to remove the activated carbon, wherein an adsorption time of the activated carbon is 20-40 min, and the amount of the activated carbon used is as follows: 0.002-0.006 g of the activated carbon is used for adsorbing every 1 mL of the material.

In a second aspect, the present disclosure provides use of the method for controlling impurities in a preparation process for an L-arabinose product.

In some embodiments, the present disclosure provides a preparation process for an L-arabinose product, which comprises step (I-a) and step (I-b) as defined above.

According to an embodiment of the present disclosure, the preparation process comprises the following steps:
(I) subjecting an L-arabinose starting raw material to step (I-a) and optionally to step (I-b) of activated carbon treatment to obtain a clean sugar liquid;
(II) subjecting the obtained clean sugar liquid to concentration, crystallization, centrifugation, and drying.

According to an embodiment of the present disclosure, step (I-a) and step (I-b) are as defined above. In some embodiments, step (II) is selected from the following step (II-a): adding a portion of the clean syrup obtained in step (I) (e.g., about half of the clean syrup) to an evaporative crystallizer, concentrating the sugar liquid to a sugar degree of 55-65% Brix, adding the remaining clean syrup in a fed-batch manner after adding a proper amount of L-arabinose as a crystal seed, and continuing concentration after the fed-batch addition is completed.

According to an embodiment of the present disclosure, the evaporative crystallizer may be selected from a conventional crystallization reactor such as an evaporative crystallization tank.

According to an embodiment of the present disclosure, in step (II-a), the sugar liquid is concentrated preferably to a sugar degree of 55-60% Brix, more preferably to a sugar degree of 58-60% Brix; after the fed-batch addition is completed, concentration continues until a solid-liquid mixture concentration is 75% to 85%.

According to an embodiment of the present disclosure, in step (II-a), during the fed-batch addition, supersaturation of the sugar liquid is controlled at 1.01-1.19, preferably at 1.01-1.10, and more preferably at 1.03-1.08.

In some embodiments, step (II) is selected from the following step (II-b): adding the clean syrup obtained in step (I) to an evaporative crystallizer for concentration, adding a proper amount of an L-arabinose raw material as a crystal seed when the syrup is concentrated to a sugar degree of 55% Brix to 65% Brix, and cooling at a rate of 0.5-3 °C per hour.

According to an embodiment of the present disclosure, in step (II-b), preferably, the sugar liquid is concentrated to a sugar degree of 58-60% Brix; preferably, the cooling rate is 0.5-2.5 °C per hour, such as 0.5 °C per hour, 1.0 °C per hour, 1.5 °C per hour, or 2.0 °C per hour. In some embodiments, the cooling rate is 0.5-2 °C per hour; in some embodiments, the cooling rate is 0.5-1.5 °C per hour.

In some embodiments, step (II-b) is selected from the following step (II-b1): removing the activated carbon by filtration, adding the obtained material to an evaporative crystallizer for concentration, adding a proper amount of an L-arabinose raw material as a crystal seed when the material is concentrated to a sugar degree of 55% Brix to 65% Brix, and cooling at a rate of 0.5-3 °C per hour. According to an embodiment of the present disclosure, in step (II-b1), preferably, the sugar liquid is concentrated to a sugar degree of 58-60% Brix; preferably, the cooling rate is 0.5-2.5 °C per hour, such as 0.5 °C per hour, 1.0 °C per hour, 1.5 °C per hour, or 2.0 °C per hour. In some embodiments, the cooling rate is 0.5-2 °C per hour; in some embodiments, the cooling rate is 0.5-1.5 °C per hour.

According to an embodiment of the present disclosure, in step (II), an activated carbon treatment step may be optionally added; in this step, the activated carbon is selected from powdered activated carbon and granular activated carbon. An adsorption temperature of the activated carbon is 20-80 °C, such as 30 °C, 35 °C, 40 °C, 45 °C, 50 °C, 55 °C, 60 °C, 65 °C, 70 °C, or 75 °C; an adsorption time of the selected activated carbon is 10-100 min, preferably 20-90 min, such as 30 min, 40 min, 50 min, 60 min, 70 min, 80 min, or 90 min. An amount of the activated carbon used is as follows: 0.0005-0.010 g of the activated carbon is used for adsorbing every 1 mL of the material, for example, 0.0005 g, 0.001 g, 0.002 g, 0.003 g, 0.004 g, 0.005 g, 0.006 g, 0.007 g, 0.008 g, 0.009 g, or 0.010 g of the activated carbon is used.

According to an embodiment of the present disclosure, in step (II), after the concentration is completed, discharging and centrifugation can be performed. After the centrifugation, the obtained L-arabinose crystal can be further dried. Preferably, the crystal is dried at a temperature of 50 °C to 60 °C for a drying time of 1.5-2.5 h, such as 2 h, with a vacuum degree of -0.01 Mpa to -0.1 Mpa, such as -0.05 MPa to -0.1 MPa.

In some embodiments, step (II) is as follows: subjecting the clean sugar liquid obtained in step (I) to evaporation until a sugar degree of the sugar liquid reaches 55% Brix to 65% Brix, performing evaporative crystallization at 50 °C to 75 °C with a vacuum degree of -0.05 Mpa to -0.1 Mpa, adding the remaining clean sugar liquid in a fed-batch manner after adding a proper amount of L-arabinose as a crystal seed, continuing concentration until a solid-liquid mixture concentration is 75% to 85% after the fed-batch addition is completed, and then performing discharging and centrifugation, wherein throughout the entire fed-batch crystallization, supersaturation of the sugar liquid is controlled at 1.01 to 1.19. Further, the step comprises the following treatment: subsequently drying the L-arabinose crystal obtained by centrifugation at a drying temperature of 50 °C to 60 °C for 2 h with a vacuum degree of -0.05 Mpa to -0.1 Mpa. According to an embodiment of the present disclosure, in the obtained L-arabinose, a content of furfural is ≤5 ppm, such as ≤4.5 ppm or ≤4.0 ppm; a content of 5-hydroxymethylfurfural is ≤3.0 ppm, such as ≤2.5 ppm, ≤2.0 ppm, ≤1.5 ppm, or ≤1.0 ppm.

According to an embodiment of the present disclosure, it is understood by those skilled in the art that in step (II) (step II-a or step II-b), the crystal seed may be selected from a conventionally available L-arabinose solid raw material (e.g., a commercially available product of L-arabinose having a purity of ≥98%), and the raw material may be further sieved and crushed, for example, sieved using No. 5 and No. 6 pharmacopeia standard sieves.

According to an embodiment of the present disclosure, the preparation process comprises the following steps:
(1) adsorbing about 1-3 L of the L-arabinose starting raw material with a D301 resin, then adsorbing with a 001×7 resin, then adsorbing with a mixed resin of D301 and 001×7, and finally adsorbing with LSA-220 at a rate of about 2 BV/h, wherein in the mixed resin, the mixing volume ratio of D301 to 001×7 is 1:1 to 3:1, such as 2:1; then warming the material to 40-60 °C, adding activated carbon for adsorption for 20-40 min, and performing suction filtration to remove the activated carbon to obtain a clean syrup;
(2) subjecting the clean syrup obtained in step (1) to evaporation until a sugar degree is 55% Brix to 65% Brix, performing evaporative crystallization at 50 °C to 75 °C with a vacuum degree of -0.05 Mpa to -0.1 Mpa, adding the remaining clean syrup in a fed-batch manner after adding a proper amount of an L-arabinose raw material as a crystal seed, continuing concentration until a solid-liquid mixture concentration is 75% to 85% after the fed-batch addition is completed, and then performing discharging and centrifugation, wherein throughout the entire fed-batch crystallization, supersaturation of the sugar liquid is controlled at 1.01 to 1.19.

The present disclosure also provides a preparation process for an L-arabinose product, and in the preparation process, an L-arabinose starting raw material can be subjected only to the treatment of step (II-b) without undergoing step (I). The preparation process can remove impurities in the L-arabinose starting raw material. The impurities are selected from at least one of furfural and 5-hydroxymethylfurfural.

Specifically, the preparation process comprises the following steps:
adding an L-arabinose starting raw material to an evaporative crystallizer for concentration, adding a proper amount of an L-arabinose raw material as a crystal seed when the L-arabinose starting raw material is concentrated to a sugar degree of 55% Brix to 65% Brix, and cooling at a rate of 0.5-3 °C per hour.

According to an embodiment of the present disclosure, in the step, preferably, the sugar liquid is concentrated to a sugar degree of 58-60% Brix; preferably, the cooling rate is 0.5-2.5 °C per hour, such as 0.5 °C per hour, 1.0 °C per hour, 1.5 °C per hour, or 2.0 °C per hour. In some embodiments, the cooling rate is 0.5-2 °C per hour; in some embodiments, the cooling rate is 0.5-1.5 °C per hour.

According to an embodiment of the present disclosure, the L-arabinose starting raw material is defined as the "L-arabinose starting raw material" used in step (I-a) above. The crystal seed is as defined in step (II) above and may be selected from a conventionally available L-arabinose solid raw material (e.g., a commercially available product of L-arabinose having a purity of ≥98%), and the raw material may be further sieved and crushed, for example, sieved using No. 5 and No. 6 pharmacopeia standard sieves.

According to an embodiment of the present disclosure, preferably, the L-arabinose starting raw material is selected from an L-arabinose-containing syrup. A sugar degree of the L-arabinose-containing syrup is 20-90% Brix, for example, the sugar degree is 20% Brix, 25% Brix, 30% Brix, 35% Brix, 40% Brix, 45% Brix, 50% Brix, 55% Brix, 60% Brix, 65% Brix, 70% Brix, 75% Brix, 80% Brix, 85% Brix, or 90% Brix; in some embodiments, the sugar degree of the L-arabinose-containing syrup is 40-50% Brix.

According to an embodiment of the present disclosure, in the preparation process, an activated carbon treatment step may be optionally added; in this step, the activated carbon is selected from powdered activated carbon and granular activated carbon. An adsorption temperature of the activated carbon is 20-80 °C, such as 30 °C, 35 °C, 40 °C, 45 °C, 50 °C, 55 °C, 60 °C, 65 °C, 70 °C, or 75 °C; an adsorption time of the selected activated carbon is 10-100 min, preferably 20-90 min, such as 30 min, 40 min, 50 min, 60 min, 70 min, 80 min, or 90 min. An amount of the activated carbon used is as follows: 0.0005-0.010 g of the activated carbon is used for adsorbing every 1 mL of the material, for example, 0.0005 g, 0.001 g, 0.002 g, 0.003 g, 0.004 g, 0.005 g, 0.006 g, 0.007 g, 0.008 g, 0.009 g, or 0.010 g of the activated carbon is used.

According to an embodiment of the present disclosure, the preparation process comprises the following steps:
concentrating a starting syrup (with a sugar degree in the range of 40-50 Brix%) by evaporation at 50 °C to 75 °C with a vacuum degree of -0.05 Mpa to -0.1 Mpa to 58-60% Brix, adding a proper amount of L-arabinose as a crystal seed, and then cooling for crystallization at a cooling rate of about 2 °C/h.

According to an embodiment of the present disclosure, in the preparation process, after the crystallization by cooling is completed, discharging and centrifugation can be performed. After the centrifugation, the obtained L-arabinose crystal can be further dried. Preferably, the crystal is dried at a temperature of 50 °C to 60 °C for a drying time of 1.5-2.5 h, such as 2 h, with a vacuum degree of -0.01 Mpa to -0.1 Mpa, such as -0.05 MPa to -0.1 MPa.

According to an embodiment of the present disclosure, in the obtained L-arabinose, a content of furfural is ≤5 ppm, such as ≤4.5 ppm or ≤4.0ppm; a content of 5-hydroxymethylfurfural is ≤3.0 ppm, such as ≤2.5 ppm, ≤2.0 ppm, ≤1.5 ppm, or ≤1.0 ppm.

In a third aspect, the present disclosure provides a product obtained by the method for controlling impurities according to the first aspect.

According to an embodiment of the present disclosure, in the product, a content of furfural is ≤5 ppm, such as ≤4.5 ppm or ≤4.0ppm; a content of 5-hydroxymethylfurfural is ≤3.0 ppm, such as ≤2.5 ppm, ≤2.0 ppm, ≤1.5 ppm, or ≤1.0 ppm.

In a fourth aspect, the present disclosure provides an L-arabinose product obtained by the preparation process according to the second aspect.

According to an embodiment of the present disclosure, in the obtained L-arabinose, a content of furfural is ≤5 ppm, such as ≤4.5 ppm or ≤4.0ppm; a content of 5-hydroxymethylfurfural is ≤3.0 ppm, such as ≤2.5 ppm, ≤2.0 ppm, ≤1.5 ppm, or ≤1.0 ppm.

In a fifth aspect, the present disclosure provides L-arabinose, and in the L-arabinose, a content of furfural is ≤5.0 ppm, such as ≤4.5 ppm or ≤4.0ppm; a content of 5-hydroxymethylfurfural is ≤3.0 ppm, such as ≤2.5 ppm, ≤2.0 ppm, ≤1.5 ppm, or ≤1.0 ppm.

### Beneficial Effects

The method and use provided by the present disclosure can effectively control the content of impurities such as 5-hydroxymethylfurfural, furfural, and the like in L-arabinose products, and in particular, they enables impurity removal from the source and the beginning of the upstream process to reduce the risk of toxic and side effects of the impurities, thereby being beneficial to improving the quality and intake safety of L-arabinose products.

### DETAILED DESCRIPTION

The technical solutions of the present disclosure are further described in detail below with reference to specific examples. It will be appreciated that the following examples are merely exemplary illustrations and explanations of the present disclosure and should not be construed as limiting the claimed scope of the present disclosure. All techniques implemented on the basis of the content described above of the present disclosure fall within the claimed scope of the present disclosure.

Unless otherwise stated, the raw materials and reagents used in the following examples are all commercially available products or can be prepared by using known methods.

The "syrup" or "raw material syrup" in the following examples refers to an L-arabinose-containing syrup. The raw materials were all commercially available products.

### Example 1. Effect of Activated Carbon Treatment on Impurity Removal Rate

Experimental materials and instruments: syrup (Zhejiang Huakang Pharmaceutical Co., Ltd., 50% Brix to 70% Brix); activated carbon (Nanping Yuanli); refractometer ATAGO PAL-α; furfural and 5-hydroxymethylfurfural detection (HPLC); microporous filter membrane (Shanghai Xingya Purification Material Factory).

1.1. A raw material syrup was treated with purified water to prepare a syrup with a sugar concentration of 32.2% Brix. The syrup was aliquoted into conical flasks (200 mL each, four flasks in total), and 0.8 g of activated carbon (4‰ activated carbon by syrup volume) was added to each flask. The mixtures were incubated in a water bath at 30 °C, 50 °C, 60 °C, and 75 °C for 30 min, respectively, followed by sequential filtration through 0.45 µm and 0.22 µm microporous filter membranes. Samples were then taken for detection. The experimental results are shown in Table 1.

**Table 1. Effect of adsorption temperature on furfural removal rate**

| **Adsorption temperature °C** | **30 °C** | **50 °C** | **60 °C** | **75 °C** |
|---|---|---|---|---|
| Furfural in the raw material syrup ng/mL | 151297 | | | |
| Furfural after treatment ng/mL | 111827 | 106831 | 112868 | 93550 |
| Furfural removal rate% | 26.09 | 29.39 | 25.40 | 38.17 |
| 5-Hydroxymethylfurfural in the raw material syrup ng/mL | 78444 | | | |
| 5-Hydroxymethylfurfural after treatment | 59775 | 66405 | 68739 | 73287 |
| 5-Hydroxymethylfurfural removal rate% | 23.8 | 15.35 | 12.37 | 6.57 |
| OD₄₀₀ value of the raw material syrup | 4.8 | | | |
| OD₄₀₀ value after treatment | 1.27 | 1.161 | 1.218 | 1.17 |
| Syrup decolorization rate% | 73.54 | 75.81 | 74.53 | 75.63 |

1.2. A raw material syrup was treated with purified water to prepare a syrup with a sugar degree of 32.1% Brix. The syrup was aliquoted into conical flasks (600 mL each, four flasks in total), and 0.3 g, 1.2 g, 2.4 g, and 4.8 g of activated carbon (0.5‰, 2‰, 4‰, and 8‰ activated carbon by syrup volume) were added to the flasks, respectively. The mixtures were incubated in a water bath at 60 °C for 60 min, followed by sequential filtration through 0.45 µm and 0.22 µm microporous filter membranes. Samples were then taken for detection. The experimental results are shown in Table 2.

**Table 2. Effect of amount of activated carbon used on furfural removal rate**

| **Amount of activated carbon used** | **0.5‰** | **2‰** | **4‰** | **8‰** |
|---|---|---|---|---|
| Furfural in the raw material syrup ng/mL | 151796 | | | |
| Furfural after adsorption ng/mL | 112383 | 113869 | 101743 | 81227 |
| Furfural removal rate% | 25.96 | 24.99 | 32.97 | 46.49 |
| 5-Hydroxyfurfural in the raw material syrup ng/mL | 78235 | | | |
| 5-Hydroxyfurfural after adsorption ng/mL | 72033 | 69484 | 63890 | 52295 |
| 5-Hydroxymethylfurfural removal rate% | 7.93 | 11.19 | 18.34 | 33.16 |
| Decolorization rate% | 29.38 | 59.50 | 77.56 | 87.20 |

1.3. A raw material syrup was prepared into a syrup with a sugar degree of 32.1% Brix. The syrup was aliquoted into conical flasks (200 mL each, three flasks in total), and 0.8 g of activated carbon (4‰ activated carbon by syrup volume) was added to each flask. The mixtures were incubated in a water bath at 60 °C for 30 min, 60 min, and 90 min, respectively, followed by sequential filtration through 0.45 µm and 0.22 µm microporous filter membranes. Samples were then taken for detection. The experimental results are shown in Table 3.

**Table 3. Effect of adsorption time on furfural removal rate**

| **Adsorption time** | **30 min** | **60 min** | **90 min** |
|---|---|---|---|
| Furfural in the raw material syrup ng/mL | 151796 | | |
| Furfural after adsorption ng/mL | 104940 | 95728 | 98616 |
| Furfural removal rate% | 30.87 | 36.94 | 35.03 |
| 5-Hydroxymethylfurfural in the raw material syrup ng/mL | 78235 | | |
| 5-Hydroxymethylfurfural after adsorption ng/mL | 62282 | 63889 | 63429 |
| 5-Hydroxymethylfurfural removal rate% | 20.39 | 18.34 | 18.93 |
| Decolorization rate% | 77.54 | 78.04 | 78.81 |

As can be seen from Table 3, when the treatment time of activated carbon reached 90 min, the adsorption capacity for impurities could not be further improved, and it was meaningless to further extend the adsorption time.

As can be seen from Tables 1 to 3, the removal rates of furfural and 5-hydroxymethylfurfural under different adsorption time, adsorption temperatures, and amounts of activated carbon used were all less than 50%. Even when the amount of activated carbon used reached 8‰ (approximately equivalent to 2.5% of the dry matter amount) and the decolorization rate reached 87%, the removal rates of furfural and 5-hydroxymethylfurfural were 32.97% and 46.49%, respectively.

### Example 2. Effect of Adsorption Resin on Impurity Removal Rate

Experimental materials and instruments: syrup (Thomson Biotech (Xiamen) Pte., Ltd., 30% Brix to 50% Brix); refractometer ATAGO PAL-α; furfural and 5-hydroxyfurfural detection (HPLC); resins (Xi'an Sunresin and Jiangsu Suqing).

### 2.1. Preliminary screening of adsorption resins

Experimental procedures: 25 g of a 32.4% Brix syrup was added to 1.0 g of a resin. The mixture was shaken on a shaker at normal temperature for 1.5 h at a shaking speed of 1000 rpm. After the resin was filtered out, the syrup sample was taken for detection. The detection results are shown in Table 4.

**Table 4. Preliminary screening results for adsorption resins**

| Resin type | Furfural **ng/mL** | 5-Hydroxymethylfurfural **ng/mL** | Furfural removal rate% | 5-Hydroxymethylfurfural removal rate% |
|---|---|---|---|---|
| Starting syrup (No resin added) | 16351 | 1020 | - | - |
| Sunresin LSA-220 | 2798 | 374 | 82.89 | 63.33 |
| Suqing D309 | 5385 | 903 | 67.07 | 11.47 |
| Suqing SQD-96 | 2254 | 756 | 86.21 | 25.88 |
| Suqing D301-M | 13247 | 839 | 18.98 | 17.75 |
| Suqing D301 | 15320 | 946 | 6.31 | 7.25 |
| Suqing D319 | 2629 | 1004 | 83.92 | 1.56 |
| Sunresin LSA-20 | 4854 | 756 | 70.31 | 25.83 |
| Sunresin Lx-10B | 3587 | 814 | 78.06 | 20.19 |

As can be seen from the detection results, the removal effect of individual use of Suqing D301-M or Suqing D301 on furfural was significantly poor, while, for example, the removal effects of Sunresin LSA-220, Suqing SQD-96, D319, Sunresin LSA-20, and Sunresin Lx-10B on furfural were relatively good. In particular, the removal effect of Sunresin LSA-220 on both furfural and 5-hydroxymethylfurfural was the best.

### 2.2. Removal of impurities by resin adsorption

Experimental materials: raw material syrup (Thomson Biotech (Xiamen) Pte., Ltd., 30% Brix to 50% Brix);
resin type: Sunresin LSA-220, macroporous adsorption resin (Xi'an Sunresin); Suqing SQD-96, styrenic macroporous weak base resin (Jiangsu Suqing).

The column was loaded by the wet method at a column loading amount of 130 mL (i.e., a volume of 1 BV), and the feed flow rate was about 2 BV/h. The sampling order is shown in the table.

**Table 5. Removal effect of resin LSA-220 on furfural and 5-hydroxymethylfurfural**

| **LSA-220** | **Furfural content ng/mL** | **5-Hydroxymethylfurfural content ng/mL** | Absorbance OD₄₀₀ |
|---|---|---|---|
| Starting syrup | 169685 | 73715 | 5.12 |
| 0-4 times BV | 22 | Not detected | 0.248 |
| 4-8 times BV | 714 | 554 | 0.690 |
| 9-11 times BV | 341 | 705 | 0.517 |
| 11-15 times BV | 1975 | 8058 | 0.732 |
| 16-19 times BV | 12270 | 16960 | 1.423 |
| 20-23 times BV | 21538 | 29906 | 2.128 |

| | | | |
|---|---|---|---|
| **Note: BV is the column bed volume.** | | | |

**Table 6. Removal effect of resin SQD-96 on furfural and 5-hydroxymethylfurfural in raw materials**

| **SQD-96** | **Furfural content ng/mL** | **5-Hydroxymethylfurfural content ng/mL** | Absorbance OD**₄₀₀** |
|---|---|---|---|
| Starting syrup | 179059 | 78126 | 4.044 |
| 1-4 times BV | 1020 | Less than 11.8 | 1.194 |
| 5-8 times BV | 2093 | 2913 | 2.460 |
| 9-12 times BV | 4851 | 7584 | 3.324 |
| 13-14 times BV | 3443 | 6142 | 3.336 |

### 2.3. Effects of different sugar degrees on resin adsorption

Experimental instruments and materials: syrup (Zhejiang Huakang Pharmaceutical Co., Ltd., 50% Brix to 70% Brix); refractometer ATAGO PAL-α; furfural and 5-hydroxyfurfural detection (HPLC); resins (Xi'an Sunresin).

Experimental procedures: 30 g of syrups with different sugar degrees (the sugar degrees could be further adjusted by treating the raw material syrup with purified water) and 2 g of a resin were placed in a shaker (1000 rpm) at normal temperature for adsorption for 1.5 h. Samples were then separately taken for detection.

**Table 7. Effects of different sugar degrees on impurity adsorption**

| **LSA-220** | **Furfural content ng/mL** | **Furfural removal rate%** | **5-Hydroxymethylfurfural content ng/mL** | **5-Hydroxymethylfurfural removal rate%** |
|---|---|---|---|---|
| Syrup 40% Brix before adsorption | 76425 | - | 74047 | - |
| Syrup 40% Brix after adsorption | 7013 | 90.82 | 14224 | 80.79 |
| Syrup 50% Brix before adsorption | 98707 | - | 94927 | - |
| Syrup 50% Brix after adsorption | 10692 | 89.17 | 21121 | 77.75 |
| Syrup 60% Brix before adsorption | 105955 | - | 104221 | - |
| Syrup 60% Brix after adsorption | 21729 | 79.49 | 43321 | 58.43 |

As can be seen from Table 7, when the sugar degree was controlled within 50% brix, the adsorption rates of furfural and 5-hydroxymethylfurfural were both not less than 75%, and when the sugar degree was controlled within 40% brix, the effect of resin adsorption was the most ideal. When the sugar degree was controlled at 60% brix, the adsorption rate of the resin to 5-hydroxymethylfurfural was less than 60%.

### 2.4. Impurity removal effect of LSA-220

Experimental materials: raw material syrup (Thomson Biotech (Xiamen) Pte., Ltd., 30% Brix to 50% Brix);
resin type: Sunresin LSA-220.

The column was loaded by the wet method. A chromatographic column with a caliber of 2.5 cm was loaded with 500 mL of a Sunresin **LSA-220** resin by the wet method, and the column flow rate was 2 BV/h. The order of fraction collection at normal temperature is shown in Table 8.

**Table 8. Adsorption of furfural and 5-hydroxymethylfurfural by resin in batches**

| **LSA-220** | **Furfural content ng/mL** | **5-Hydroxymethylfurfural content ng/mL** | **%Brix** |
|---|---|---|---|
| Starting syrup | 180856 | 21056 | 42.9 |
| 1-4 times BV | 32 | Not detected | 41.6 |
| 5-8 times BV | 41 | 61 | 43.0 |
| 9-12 times BV | 50 | 70 | 42.9 |
| 13-16 times BV | 61 | 458 | 42.9 |
| 17-20 times BV | 73 | 2012 | 42.9 |

### 2.5. Impurity removal effect of resin combination

Experimental materials: raw material syrup (Thomson Biotech (Xiamen) Pte., Ltd., 30% Brix to 50% Brix);
resin type: Jiangsu Suqing D-301, Jiangsu Suqing 001×7.

The column was loaded by the wet method. 3 chromatographic columns with a caliber of 2.5 cm were taken, and a starting syrup sequentially passed through the columns loaded with 150 mL of a Suqing **D301 resin, 75 mL of a Suqing 001×7 resin, and 150 mL of a mixed resin** (the volume ratio of D301 to 001×7 was 2:1) by the wet method at a column flow rate of 2 BV/h.

**Table 9. Adsorption of furfural and 5-hydroxymethylfurfural by resin combination**

| **001×7-D301-mix** | **Furfural content ng/mL** | **5-Hydroxymethylfurfural content ng/mL** | **%Brix** |
|---|---|---|---|
| Starting syrup | 180856 | 21056 | 42.9 |
| 1-4 times BV | 67 | 133 | 28.3 |
| 5-8 times BV | 357 | 596 | 45.5 |
| 9-12 times BV | 1982 | 1097 | 42.7 |
| 13-16 times BV | 14537 | 7867 | 43.2 |

### Example 3. Study on Impurity Content in Process

**3.1. Experimental materials and instruments:** syrup (Thomson Biotech (Xiamen) Pte., Ltd.); refractometer ATAGO PAL-α; furfural and 5-hydroxyfurfural detection (HPLC); L-arabinose crystal seed (i.e., powder obtained by grinding and sieving an L-arabinose raw material by No. 5 and No. 6 pharmacopeia standard sieves, the raw material purchased from Thomson Biotech (Xiamen) Pte., Ltd.).

### 3.2. Experimental procedures:

Starting syrup profile of crystallization batch No. 1213: 180856 ng/mL of furfural, 21056 ng/mL of 5-hydroxymethylfurfural, and a sugar degree of 42.9% brix; concentrated to about 800 mL, and fed-batch addition of about 1200 mL. The dry matter feeding amount was about 1055 g.

Starting syrup profile of crystallization batch No. 1215: 24074 ng/mL of furfural, 885 ng/mL of 5-hydroxymethylfurfural, and a sugar degree of 43.9% brix; concentrated to about 800 mL, and fed-batch addition of about 1200 mL. The dry matter feeding amount was about 1073 g.

The starting syrup was concentrated by evaporation to 55% Brix to 65% Brix, and the evaporative crystallization was performed at 50 °C to 75 °C with a vacuum degree of -0.05 Mpa to -0.1 Mpa. After 1.5 g of L-arabinose was added as a crystal seed, the remaining starting syrup was added in a fed-batch manner. After the fed-batch addition was completed, the concentration continued until the solid-liquid mixture concentration was 75% to 85%, and then discharging and centrifugation were performed. Throughout the entire fed-batch crystallization, supersaturation of the sugar liquid was controlled at 1.01 to 1.19.

The arabinose crystal obtained by centrifugation was dried at a drying temperature of 50 °C to 60 °C for 2 h with a vacuum degree of -0.05 Mpa to -0.1 Mpa.

**Table 10-1. Content of impurities in samples in process**

| Crystallization batch No. | Sampling type | Furfural content | 5-Hydroxymethylfurfural content | Sugar degree% Brix |
|---|---|---|---|---|
| 1213 | Starting syrup | 24074 ng/mL | 885 ng/mL | 43.9% |
| | Centrifugation mother liquor | 2834 ng/mL | 1992 ng/mL | - |
| | Crystallization sample | 0.23 ppm | 1.82 ppm | |
| 1215 | Starting syrup | 180856 ng/mL | 21056 ng/mL | 42.9% |
| | Centrifugation mother liquor | 3977 ng/mL | 37886 ng/mL | - |
| | Crystallization sample | 0.2 ppm | 2.17 ppm | - |

As can be seen from Table 10-1, the contents of 5-hydroxymethylfurfural in the two batches without resin treatment were 885 ng/mL and 21056 ng/mL, respectively.

### 3.3. Experimental procedures:

About 1000 mL of a starting syrup (with a sugar degree in the range of 40-50% Brix) was concentrated by evaporation at 50 °C to 75 °C with a vacuum degree of -0.05 Mpa to -0.1 Mpa to 60% Brix and cooled for crystallization in a water bath at 65 °C. After 0.75 g of L-arabinose was added as a crystal seed, the mixture was cooled for crystallization at a cooling rate of about 2 °C/h. Discharging and centrifugation were performed when the mixture was cooled to 31 °C. The arabinose crystal obtained by centrifugation was dried at a drying temperature of 50 °C to 60 °C for 2 h with a vacuum degree of -0.05 Mpa to -0.1 Mpa.

Activated carbon could be used for adsorption, and the conditions were as follows: using about 0.8 g of activated carbon for adsorption at 50 °C for 30 min.

In the crystallization process, the impurity content in each sample was tested, as shown in Table 10-2 below.

**Table 10-2. Content of impurities in samples in process**

| Crystallization batch No. | Sampling type | Furfural content | 5-Hydroxymethylfurfural content |
|---|---|---|---|
| 0104 | Starting syrup (sugar degree of 43.9% Brix) | 24074 ng/mL | 885 ng/mL |
| | Centrifugation mother liquor | 2759 ng/mL | 1868 ng/mL |
| | Crystallization sample | Not detected | Not detected |
| Crystallization batch No. | Sampling type | Furfural content | 5-Hydroxymethylfurfural content |
| 0111 | Starting syrup (sugar degree of 45.4% Brix) | 180856 ng/mL | 21056 ng/mL |
| | Activated carbon adsorption | 9112 | 5555 |
| | Centrifugation mother liquor | 11168 | 8048 |
| | Crystallization sample | Not detected | 0.63 ppm |
| Crystallization batch No. | Sampling type | Furfural content | 5-Hydroxymethylfurfural content |
| 0113 | Starting syrup (sugar degree of 48% Brix) | 78082 ng/mL | 9830 ng/mL |
| | Centrifugation mother liquor | 23477 ng/mL | 14360 ng/mL |
| | Crystallization sample | Not detected | 0.37 ppm |
| Crystallization batch No. | Sampling type | Furfural content | 5-Hydroxymethylfurfural content |
| 0114 | Starting syrup (sugar degree of 48% Brix) | 21670 ng/mL | 3085 ng/mL |
| | Centrifugation mother liquor | 44 ng/mL | 1734 ng/mL |
| | Crystallization sample | Not detected | Not detected |

As can be seen from Table 10-2 above, the cooling crystallization process in 3.3 had a certain impurity removal effect on furfural and 5-HMF.

### Example 4. Effect of Adopting Resin Adsorption on Impurities in Preparation Process

Starting material syrup profile of crystallization batch No. 1221 and crystallization batch No. 1229: 180856 ng/mL of furfural, 21056 ng/mL of 5-hydroxymethylfurfural, and a sugar degree of 42.9% brix.

**4.1. Experimental materials and instruments:** syrup (Thomson Biotech (Xiamen) Pte., Ltd.); refractometer ATAGO PAL-α; furfural and 5-hydroxyfurfural detection (HPLC); resins: Sunresin LSA-220 (Xi'an Sunresin), Jiangsu Suqing D-301, and Jiangsu Suqing 001×7; activated carbon (Nanping Yuanli); L-arabinose crystal seed (i.e., powder obtained by grinding and sieving an L-arabinose raw material by No. 5 and No. 6 pharmacopeia standard sieves, the raw material purchased from Thomson Biotech (Xiamen) Pte., Ltd.).

### 4.2. Experimental procedures:

(1) About 2 L of a raw material syrup sequentially passed through columns loaded with 150 mL of a Suqing D301 resin, 75 mL of a 001×7 resin, and 150 mL of a mixed resin (the volume ratio of D301 to 001×7 was 2:1) by the wet method at a rate of about 2 BV/h, followed by passing through 150 mL of a Sunresin LSA-220. The syrup was then warmed to 50 °C, and 8 g of activated carbon was added for adsorption for 30 min. After the activated carbon was removed by suction filtration, a clean syrup was obtained.
(2) The clean sugar liquid obtained in step (1) was evaporated to a sugar degree of 55% Brix to 65% Brix, and the evaporative crystallization was performed at 50 °C to 75 °C with a vacuum degree of - 0.05 Mpa to -0.1 Mpa. After 1.5 g of L-arabinose was added as a crystal seed, the remaining clean sugar liquid was added in a fed-batch manner. After the fed-batch addition was completed, the concentration continued until the solid-liquid mixture concentration was 75% to 85%, and then discharging and centrifugation were performed. Throughout the entire fed-batch crystallization, supersaturation of the sugar liquid was controlled at 1.01 to 1.19.
(3) The arabinose crystal obtained by centrifugation was dried at a drying temperature of 50 °C to 60 °C for 2 h with a vacuum degree of -0.05 Mpa to -0.1 Mpa.

**Table 11. Content of impurities in samples in process (adopting resin adsorption)**

| **Crystallization batch No.** | **Sampling type** | **Furfural content** | **5-Hydroxymethylfurfural content** | **Sugar degree% Brix** |
|---|---|---|---|---|
| 1221 | Clean syrup | 54 ng/mL | 33 ng/mL | 30 |
| | Centrifugation mother liquor (semi-solid) | 0.29 ppm | Not detected | - |
| | Crystallization sample | Not detected | Not detected | - |
| 1229 | Clean syrup | 61 ng/mL | 458 ng/mL | 42 |
| | Centrifugation mother liquor (semi-solid) | 0.38 ppm | 1.4 ppm | - |
| | Crystallization sample | Not detected | Not detected | - |

As can be seen from Table 11, when the concentration of 5-hydroxymethylfurfural in the clean syrup was controlled at 458 ng/mL or less, both 5-hydroxymethylfurfural and furfural in the arabinose crystallization samples were not detected. The adsorption resin adopted in the present disclosure can further effectively control furfural and 5-hydroxymethylfurfural and reduce the risk of possible toxic and side effects.

### Example 5. Bowel Cleansing Effect of L-Arabinose as Drug for Bowel Preparation

Experimental materials: L-arabinose, selected from the L-arabinose crystallization sample with batch No. 1221 in the example, prepared into a 0.4 g/mL solution with purified water; PEG (Shutaiqing), prepared into a 0.521 g/mL PEG solution with purified water; SPF-grade rats (Zhejiang Vital River Laboratory Animal Technology Co., Ltd.).

5.1. Experimental animals and feeding thereof: SPF (specific pathogen free)-grade rats (200±10 g), 6 weeks old; normal feed was D12450-B feed: 67.35 wt% carbohydrates, 19.2 wt% crude protein, and 4.3 wt% fat. The temperature of the room was kept at 24 °C to 26 °C with humidity at 40% to 60%.

### 5.2. Experimental method and procedures:

The SPF-grade rats were randomly divided into a blank control group, a positive control group, and a treatment group, 10 animals per group, half being male and half female. The dosage administered to each group is shown in Table 12 below. Administration method: Animals in each group were fasted (water was accessible) starting at 22:00 the night before experiment, were each given 1/2 the calculated amount of drug by intragastrical administration at 7:00 and 9:00 on the day of experiment, were each given 2.0 mL of purified water at 12:00 and 13:00, and were dissected 3.5 h after the last administration.

**Table 12. Rat bowel preparation experiment**

| Group | Treatment | Administration dosage (g/kg) | Administration volume (mL/kg) |
|---|---|---|---|
| Negative control group | Purified water | / | 42 |
| Positive control group | PEG | 21.9 | 42 |
| Treatment group | L-Arabinose with batch No. 1221 | 9 | 22.5 |

| | | | |
|---|---|---|---|
| Note: The test substance was administered to each administration group in 2 doses. | | | |

### 5.3. Experimental results:

(1) Watery and loose stools were observed in both the positive control group and the treatment group before the dissection.
(2) It was observed that no residue was left in the rectum and colon in the positive control group and the treatment group after the dissection, which indicates good bowel cleansing effects. Apparently, it took only a dosage that was 41% of that administered to the positive control group for the L-arabinose treatment group to achieve the same effect. The treatment group has a significant advantage in terms of administration dosage.

### Example 6. Therapeutic Effect of L-Arabinose on Constipation Model Rats

Experimental materials: L-arabinose, selected from the arabinose crystallization sample with batch No. 1229 in the example.

### 6.1. Experimental preparation

Modeling procedure: modeling with compound diphenoxylate.

Experimental drugs and reagents: activated carbon (5%); a 0.4 g/mL aqueous solution prepared from an arabinose drug.

Positive control: PEG (Shutaiqing). SPF-grade rats (Zhejiang Vital River Laboratory Animal Technology Co., Ltd.).

### 6.2. Experimental procedures:

Except for the normal group, the successfully modeled rats were randomly divided into 5 groups, including a model group, a low dose group, a medium dose group, a high dose group, and a positive control group, 8 animals per group. Each rat was housed individually in separate cages, and operations were conducted fixedly at 10:00-12:00 every day. The normal group and the model group were gavaged with 0.2 mL of purified water. The positive control group was gavaged with 0.133 mL of a mosapride solution, and the low dose group, the medium dose group, and the high dose group were gavaged with 0.01 mL, 0.02 mL, and 0.04 mL of the test drug, respectively, with the volume adjusted to 0.2 mL using purified water. The rats had free access to food and water. The administration was performed for 7 consecutive days (times).

For the first administration, the drug with 5% activated carbon (based on 0.2 g × 5%) added was intragastrically administered to the treatment group, and 0.2 mL of purified water with activated carbon added was intragastrically administered to the normal group and the model group. The time for the first dark stools was recorded.

The rats were intragastrically administered once daily. On day 7 after the continuous administration, the stools were collected using a sterile centrifuge tube and stored at -100 °C. The collected stools were dried at 105 °C to a constant weight, and the water content of the stools was calculated according to the formula: water content of stools = (1 - mass after drying/mass before drying) × 100%.

The rats were intragastrically administered once daily for 14 days and then fasted for 12 h. 1 h after the last administration, each group of animals was gavaged with 0.15 mL of 5% activated carbon, and 30 min later, the rats were anesthetized with an intraperitoneal injection of 10% chloral hydrate. After anesthesia was completed, the abdominal operation was immediately performed. The intestinal canal from pylorus to ileocecal junction was cut off and placed on a glass plate. The length of the intestinal tract traversed by the ink and the total length of the intestinal tract were measured. The intestinal propulsion rate was the ratio of the two. The total length from pylorus to ileocecal junction and the distance from pylorus to the front of the carbon ink were measured, and the carbon ink propulsion percentage was calculated. Intestinal propulsion rate = distance from front of carbon ink to pylorus/full length of small intestine × 100%.

**Table 13. Indicators of mice after drug intervention ( x̅±sd, n = 8)**

| **Category** | **Dose kg/d** | **Small intestine propulsion rate%** | **Water content of stools%** | **Time for first dark stools min** |
|---|---|---|---|---|
| Normal group | 0 | 78%±2.5* | 51%±1.9* | 80±7.8 |
| Low dose | 0.20 g | 75%±3.1* | 54%±1.8* | 76±8.5* |
| Medium dose | 0.40 g | 80%±2.7* | 59%±2.7* | 64±6.2* |
| High dose | 0.80 g | 83%±2.8* | 61%±2.4* | 63±5.7* |
| Model group | 0 | 62%±3.3 | 40%±1.7 | 158±10.6 |
| Positive control | 2.0 mg | 82%±4.4* | 57%±0.9* | 67±8.8* |

After t-test, the small intestinal propulsion rate, the water content of stools, and the time for the first dark stools of the three dose groups were significantly different from those of the model group (*p < 0.01). From the low dose group to the high dose group, the small intestinal propulsion rate tended to increase, but there was no significant difference between the groups, indicating that the effect of the drug intervention was significant, the water content in the mice increased significantly, and the defecation was smooth. The administration group showed no significant difference compared to the positive control group, indicating equivalent efficacy intestinal propulsion rate and fecal water content.

The embodiments of the present disclosure have been described above. However, the embodiments described above are not intended to limit the present disclosure. Any modification, equivalent replacement, improvement, and the like made without departing from the spirit and principle of the present disclosure shall fall within the protection scope of the present disclosure.

## Claims

1. A method for controlling impurities, comprising:
(I-a) removing impurities in an L-arabinose starting raw material by using an adsorption resin,
wherein
the impurities are selected from at least one of furfural and 5-hydroxymethylfurfural;
the adsorption resin is selected from at least one of a macroporous adsorption resin with high specific surface area, a styrenic macroporous resin, and a styrene-divinylbenzene copolymer skeleton macroporous resin.

2. The method according to claim 1, wherein the adsorption resin is selected from at least one of a styrenic macroporous strong acid cation exchange resin and a styrenic macroporous weak base anion exchange resin; or the adsorption resin is selected from a sulfonic acid-based cation exchange resin, wherein
preferably, the sulfonic acid-based cation exchange resin is selected from a cation exchange resin having a sulfonic acid group (-SO₃H) on a styrene-divinylbenzene copolymer;
preferably, the adsorption resin is selected from at least one of LSA-220, D309, SQD-96, D301-M, D301, D319, LSA-20, Lx-10B, LX-100B, LX-20B, LSA-10, LSA-12, LSA-10B, LSA-100, LSA-210, HPD500, HPD300L, D-900, and 001×7;
more preferably, the resin is selected from at least one of LSA-220, SQD-96, D319, LSA-20, and Lx-10B;
more preferably, the resin is selected from at least one of LSA-220 and SQD-96;
more preferably, the adsorption resin is selected from a combination of D301 and 001×7.

3. The method according to claim 1 or 2, wherein the L-arabinose starting raw material is first adsorbed by a D301 resin, then adsorbed by a 001×7 resin, and finally adsorbed by a mixed resin of D301 and 001×7; in the mixed resin, a mixing volume ratio of D301 to 001×7 is 1:1 to 3:1, such as 2:1.

4. The method according to any one of claims 1-3, wherein the L-arabinose starting raw material is selected from an L-arabinose-containing syrup, and a sugar degree of the L-arabinose-containing syrup is 20-70% Brix.

5. The method according to any one of claims 1-3, wherein a sugar degree of the L-arabinose starting raw material is ≤60% Brix when passing through a column, preferably, the sugar degree is ≤50% Brix, and more preferably, the sugar degree is ≤40% Brix; preferably, in step (I-a), a column flow rate for the material is 0.5 BV/h to 4 BV/h; the column temperature is 10-50 °C; an elution solvent used is water.

6. The method according to any one of claims 1-3, further comprising step (I-b) of activated carbon treatment, wherein
preferably, in step (I-b), an adsorption temperature of the activated carbon is 20-80 °C; an adsorption time of the selected activated carbon is 10-100 min; an amount of the activated carbon used is as follows: 0.0005-0.010 g of the activated carbon is used for adsorbing every 1 mL of the L-arabinose material.

7. Use of the method for controlling impurities according to any one of claims 1-6 in a preparation process for an L-arabinose product, wherein the preparation process comprises the following steps:
(I) subjecting an L-arabinose starting raw material to step (I-a) and optionally to step (I-b) of activated carbon treatment to obtain a clean sugar liquid;
(II) subjecting the obtained clean sugar liquid to concentration, crystallization, centrifugation, and drying;
wherein step (I-a) and step (I-b) are as defined in any one of claims 1-6.

8. The use according to claim 7, wherein step (II) is selected from the following step (II-a):
adding a portion of the clean syrup obtained in step (I) (e.g., about half of the clean syrup) to an evaporative crystallizer, concentrating the sugar liquid to a sugar degree of 55-65% Brix, adding the remaining clean syrup in a fed-batch manner after adding a proper amount of L-arabinose as a crystal seed, and continuing concentration after the fed-batch addition is completed;
in step (II-a), the sugar liquid is concentrated preferably to a sugar degree of 55-60% Brix, more preferably to a sugar degree of 58-60% Brix; after the fed-batch addition is completed, concentration continues until a solid-liquid mixture concentration is 75% to 85%;
preferably, in step (II-a), during the fed-batch addition, supersaturation of the sugar liquid is controlled at 1.01-1.19;
or step (II) is selected from the following step (II-b): adding the clean syrup obtained in step (I) to an evaporative crystallizer for concentration, adding a proper amount of an L-arabinose raw material as a crystal seed when the syrup is concentrated to a sugar degree of 55% Brix to 65% Brix, and cooling at a rate of 0.5-3 °C per hour;
in step (II-b), preferably, the sugar liquid is concentrated to a sugar degree of 58-60% Brix; preferably, the cooling rate is 0.5-1.5 °C per hour; preferably, in step (II), after the concentration is completed, discharging and centrifugation can be performed; more preferably, after the centrifugation, the obtained L-arabinose crystal can be further dried;
more preferably, the crystal is dried at a temperature of 50 °C to 60 °C for a drying time of 1.5-2.5 h with a vacuum degree of -0.05 Mpa to -0.1 Mpa.

9. A preparation process for an L-arabinose product, comprising the following steps:
adding an L-arabinose starting raw material to an evaporative crystallizer for concentration, adding a proper amount of an L-arabinose raw material as a crystal seed when the L-arabinose starting raw material is concentrated to a sugar degree of 55% Brix to 65% Brix, and cooling at a rate of 0.5-3 °C per hour, wherein the L-arabinose starting raw material is selected from an L-arabinose-containing syrup, and a sugar degree of the L-arabinose-containing syrup is 40-50% Brix.

10. An L-arabinose product obtained by the preparation process according to claims 7-9.

11. The product according to claim 10, wherein a content of furfural is ≤5.0 ppm, and a content of 5-hydroxymethylfurfural is ≤3.0 ppm.
